# EUROPEAN PATENT SPECIFICATION

(11) **EP 0 794 786 B1**
(45) Date of publication and mention of the grant of the patent: **20.08.2003**
(21) Application number: 95942373.2
(22) Date of filing: 22.12.1995
(51) Int. Cl.: A61K 31/66, C07F 9/117

(54) **THE USE OF INOSITOLTRISPHOSPHATE FOR THE PREPARING OF MEDICAMENTS**
VERWENDUNG VON INOSITTRIPHOSPHAT ZUR HERSTELLUNG VON ARZNEIMITTELN
UTILISATION D'INOSITOLTRIPHOSPHATE POUR LA PREPARATION DE MEDICAMENTS

(30) Priority: 30.12.1994 SE 9404568
(43) Date of publication of application: 17.09.1997
(73) Proprietor: Bioneris AB,, 181 31 Lidingö (SE)
(72) Inventor: SIREN, Matti, Helsingfors 17 (FI)
(74) Representative: Hansen, Bernd, Dr. Dipl.-Chem.
(86) International application number: SE9501585
(87) International publication number: WO96020715

(56) References cited:
- US-A- 4 590 060
- US-A- 4 952 396

## Description

The present invention relates to the use of D-myo-inositol-1,2,6-trisphosphate for preparing of a medicament with antimetastatic effects.

Tumor diseases may be defined as a progressive series of genetic events which occur in a single clone of cells as a result of alterations in a limited number of specific genes i.e. the oncogenes and the tumor suppressor genes. Consistent chromosome aberrations are observed for example in lung, colon, and breast cancers. In this manner tumor diseases can be understood as a result of the accumulation of multiple genetic changes (E. Solomon et al., Science, 254, 1153-1160, 1991).

one severe type of tumor diseases are brain tumors or gliomas. In humans, gliomas account for over 60 % of primary intracranial neoplasms. Most commonly, gliomas arise from malignant transformations of astrocytes and according to histopathological criteria they are classified as astrocytoma (low grade glioma or glioblastoma, or high grade glioma). A glioblastoma is a tumor characterized by high cell density, cellular polymorphism, mitoses, necroses with palisading cells and prominent vascularisation with endothelial cell proliferation.

Often glioblastoma is seen as the prototypical tumor when developing new therapeutical regimes.

The progressive in vivo growth of malignant tumor cells within tumor tissue, becomes possible only after the formation of an adequate blood supply system within tumor tissue. Such a system is formed by the incorporation of existing host vessels into the tumor tissue as well as by the creation of new tumor microvessels. The vasculature network in human cancers is essential for the existence of these malignant tissue systems. The tumor vasculature network provides cancer cells with vital nutrients and also enables the removal of waste products generated by the cells. The vascularization of tumor tissue also influences positively the proliferation of tumor cells. For example, tumor cells in the vicinity of blood vessels have a greater growth rate than those tumor cells which are located further away from blood vessels.

Angiogenesis is a central part of the process of blood vessel formation. In normal physiological activities including reproduction and tissue development angiogenesis is highly regulated as it is activated for only brief periods and then completely inhibited. In many diseases, however, the regulation of angiogenic activity is loosened. In some cases angiogenic activity becomes completely unregulated and its rate is exhilarated.

Angiogenic activity is central to the proliferation of tumor cells in a human body. The often rapid development of new blood vessels through angiogenesis provides a highly effective means for this process. The new blood vessels become a gateway through which tumor cells enter the human circulatory system and eventually metastasize into distant sites such as the liver, the lungs, bones, etc. (J. Folkman, Y. Shing; J. Biol. Chem., 267, 10931-10934, 1992).

When a healthy cell is on the borderline of becoming a malignant tumor cell, the tissue which surrounds the cell must induce angiogenic vessel formation in order to attract the nourishing vasculature which the developing cells need for their growth. In such cases angiogenesis is controlled by a balance between local factors which on the one hand stimulate vasculature growth and on the other inhibit vasculature growth. In most normal tissues, the inhibitory influences of the local factors predominate the angiogenic process, and cells which are derived from such tissues generally do not stimulate angiogenesis.

In many pathological conditions, however, diseases appear to be driven by, and spread through unregulated angiogenesis. A primary example of such abnormal, unregulated angiogenic activity is the proliferation and metastasis of solid tumors.

An in vivo tumor is formed only when the host produces a stroma and the vascular network which is required for the survival and growth of the neoplastic cell population.

The stroma consists of (a) provisional fibrin gel matrix, (b) new blood vessels, (c) inflammatory cells, (d) connective tissue. Malignant growth has been shown to depend in general on complex interactions between the tumor and the immune- and homeostatic systems.

The growth of solid tumors beyond microscopic clumps of cells requires the development of a vascular network which provides the malignant cells with nutrients and oxygen. The additional nutrients and oxygen supplied through the new vasculature allows dysplastic growth of the transformed cell populations; this in turn promotes further vessel development. Differentiation, mutation, and selection of tumor cells occurs throughout this cycle, and the formation of three-dimensional tumors is the ultimate result of the process. Such tumors may be locally invasive or metastatic, or both.

A continuous remodelling of the tumor extra-cellular matrix controlled by malignant cells which are characterized by different degrees of biosynthesis and degradation, has been observed during the development of primary and metastatic tumors (Iozzo, R.V. and Cohen, I., 1993; Dvorak, H.F. et al, 1992).

Abnormal accumulation of fluid typically accompanies solid-, and particularly ascites tumor growth. The formation of ascites fluids is made possible by rapidly increasing microvascular permeability. Observations made in experimental animal studies as well as clinical human studies seem to indicate that accumulation of ascites fluid can be attributed to alterations in the permeability of the vessels which form the lining for the peritoneum and other cavities. The vessels which form the lining for the peritoneal cavity in ascites tumor-bearing guinea pigs, hamsters, and mice manifest significantly greater permeability than similar vessels in control animals. Secretion of permeability-increasing activity appears to be a common feature in tumor tissue and may contribute to the abnormal accumulation of fluid in neoplastic diseases.

All tumor tissue, solid or otherwise compact, consists of different types of cells, never of individual cells living in isolated environments.

Tumor diseases comprise more than one hundred different diseases. Each of these has between two and four stages and each can be divided into smaller, more or less critical subtypes based on histological criteria and enzymological and other findings. All of the different stages and subsets are potential targets for therapeutic treatment and combinations thereof. Such treatments include chemotherapy, hormone therapy, radiotherapy, cytokine therapy, and surgery.

It is significant to note that even though the term "chemotherapy" is generally used to denote the application of cytotoxic agents the term actually refers to over 30 quite different cytotoxic agents all of which are in common clinical use. It is also important to keep in mind that when cytotoxic agents are administered alone or in combination with one another they have extremely steep doseresponse curves. Because the dose- and response curves of most cytotoxic drugs coincide with their toxicity- and efficacy level curves, in therapy most of these agents are applied at doses which are close to their maximum level of tolerability. As a result, the harmful side effects of cytotoxic drugs are difficult to avoid. Discrepancies in individual patient's drug metabolism and dose-response relationship make the situation even more problematic.

During the past decades cytotoxic drugs have become increasingly popular as a treatment for neoplastic diseases and they have become invaluable for clinical cancer therapy. In order to combat the heterogeneity of the variable tumor cell metabolism of the neoplastic cell population, many of these therapeutic agents are frequently used in combination with one another. In cancer chemotherapy such polypharmacy has become almost a rule. Today it is also clear that many anti-cancer drugs are essentially prodrugs which require metabolic-activation in order to exert their selective cytotoxic effects.

Many cytotoxic agents are not very selective in terms of their target cells i.e. they are cytotoxic also towards normal, healthy cells.

Antineoplastic drug-therapy treatments function selectively by targeting rapidly dividing cells. However, clinical experience shows that many curable cancers grow at a relatively slow rate whereas many treatment-resistant cancers are quite rapidly spreading.

Recent research seems to suggest that one of the most significant effects of antineoplastic drugs is the induction of apoptosis in tumor cells. Apoptosis is a genetically encoded and activated cell death program which is defined by specific morphological and biochemical changes within a cell. A multitude of factors regulate apoptosis induction; these include, for example, intracellular mediators of signal transduction, the modulation of gene expression by nuclear proteins, DNA replication, and cell cycle.

Another approach for curing tumor diseases has been to develop angiosuppresive agents. However, almost all potential angiosuppresive agents have proven to be too toxic for clinical use as their tolerability in humans generally are too low.

The primary focus of cancer research is the individual cancer cell and its metabolism. However, the symbiotic relationship of a cancer cell and its surrounding environment has escaped critical attention.

To summarize there is a need for a new and effective anti-tumor agent without harmful side-effects.

Ideally a new anti-tumor agent should fulfill the following criteria: (a) it must act specifically against tumor tissue; (b) it should have no harmful side-effects; (c) a new substance should not be a cytostatica because the aim is not the destruction of a single tumor cell, but instead the blocking of the tumor tissue growth mechanism; (d) it must be able to prevent the angiogenesis of tumor tissue; (e) it must have an antimetastatic effect.

According to the present invention it has surprisingly become possible to use D-myo-inositol-1,2,6-trisphosphate for preparing of a medicament with antimetastatic effects.

In preferred embodiments of the invention the medicament is intended to be used for preventing, alleviating and combatting tumor tissue growth and angiogensis of tumor tissue.

The invention relates to the use of D-myo-inositol-1,2,6-trisphosphate for preventing, alleviating or combatting for example the following conditions:
Glioma, glioblastoma, sarcoma such as Kaposi sarcoma, prostate carcinoma', colon adenocarcinoma, pancreas carcinoma, mamma adenocarcinoma, lung carcinoma such as small cell lung carcinoma, human histiocytic lymphoma and melanoma.

The medicament is effective against the above mentioned conditions but also to other conditions of tumor growth and especially growth of solid tumors.

The medicament exerts significant antimetastatic effects without side-effects which is very beneficial for the patient.

From the European Patent No. 179439 a pharmaceutical composition comprising as a pharmaceutically active ingredient at least one isomer of inositoltrisphosphate is known. In said patent the effect of this pharmaceutical composition is shown for different areas, such as platelet aggregation.

The production of inositoltrisphosphate (IP₃) and the isolation of the different isomers thereof are disclosed in the US patent No. 4.777.134. The IP₃ isomers can also be produced by synthetic methods, chemically or enzymatically, starting with e.g. inositol and a phosphorus source. Furthermore, microbiological production methods including hybrid DNA-techniques of IP₃ are also suitable.

The structure of IP₃ and the different isomers thereof are disclosed in the US patent No. 4.735.936 and the US patent No. 4.797.390.

US 4,952,396 discloses a method for inhibiting tumor growth, which uses phytic acid, phytic salts or hydrolysates thereof.

US 4,590,060 discloses an agent facilitating liposome cellular wall transport. It further discloses a kit for use in diagnosis and therapy. Said kit may contain inositol phosphates for the purpose of increasing deoxygenation.

It is suitable that the medicament used according to the invention exists in unit dosage form. Tablets, granules or capsules are suitable administration forms for such unit dosage. Furthermore, tablets and.granules can easily be surface treated such as to provide an enteric coating to prevent an uncontrolled hydrolysis in the stomach and to bring about a desired absorption in the intestine. Other suitable administration forms are slow release and transdermal administration. A usual pharmaceutically acceptable additive, excipient and/or carrier can be included in the medicament. The tablets or granules can also contain a disintegrant which causes the tablets or the granules, respectively, to disintegrate easily in the intestine. In certain cases, especially in acute situations, it is preferable to use the unit dosage in the form of a solution for intravenous administration. In other situations suspensions comprising the compound can be preferably used as administration form.

The medicament can also consist as such of D-myo-inositol-1,2,6-trisphosphate solely without any additive, excipient or carrier.

If desired, the medicament can be free of other inositol phosphates, IP₁, IP₂, IP₄, IP₅ and IP₆. Accordingly, the D-myo-inositol-1,2,6-trisphosphate can have a purity of 90-100%, such as 93-100% or preferably 95-100%.

The medicament can consist of D-myo-inositol-1,2,6-trisphosphate, wherein said D-myo-inositol-1,2,6-trisphosphate is provided by at least one of IP₆, IP₅ or IP₄ and a degradative substance such as an enzyme suitable to form D-myo-inositol-1,2,6-trisphosphate.

It is in most cases suitable that the D-myo-inositol-1,2,6-trisphosphate used for the preparing of the medicament according to the invention is present in salt form in order not to affect the mineral balance negatively. The salt should preferably consist of a sodium, potassium, calcium, zinc or magnesium salt or a mixture of two or more of these salts.

For the above mentioned reasons it is also an advantage if the medicament contains a surplus or an extra addition of at least one pharmaceutically acceptable salt of calcium, zinc or magnesium with a mineral acid or organic acid. This is especially valuable for elderly persons who are often deficient in these minerals.

For administration to human patients appropriate dosages can routinely be determined by those skilled in this art by extension of the results obtained in animals at various dosages. The preferred dosage for humans falls within the range of 0.1 to 1000 mg, especially 0.1-200 mg D-myo-inositol-1, 2, 6-trisphosphate/day/kg body weight.

In animal experiments, no toxic effects were seen after administration of very high doses of D-myo-inositol-1, 2, 6-trisphosphate, 160 mg/kg body weight by intraperitoneal injection to mice.

The medicament usually contains 0.01-1.5g, such as 0.05-1.3g or preferably 0.1-1g of D-myo-inositol-1,2,6-trisphosphate per unit dosage.

The composition used according to the present invention contains:
D-myo-inositol-1,2,6-trisphosphate of the formula 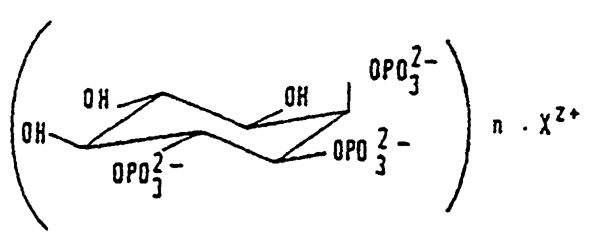 where X is hydrogen, at least one univalent, divalent or multivalent cation, or a mixture thereof, n is the number of ions, and z is the charge of the respective ion.

In the above formula n ranges between 6 to 1 inclusive and z ranges from 1 to 6 inclusive. Preferably, n is between 3 to 6 inclusive and z is 3, 2 or 1.

The invention will be further explained in the following examples without limiting it thereto. Example 1 shows the manufacturing of a solution of IP₃ for intravenous administration and Example 2 illustrates the ability of IP₃ to counteract the tumor growth of glioma tumor tissue in nude mice. Nude mice have been used extensively for the transplantation and propagation of human xenografts in order to design adequate in vivo experiments with high correlation to effects in the human body.

To further increase the prediction of animal experiments for situations in the human body the severe combined immunodeficient mouse (SCID) has been utilized as this animal allows heterotransplantation of human tumors. Example 3 demonstrates the antimetastatic effect of IP₃ in a prostate tumor experiment and example 4 shows the antiangiogenetic effect of IP₃ in a lung tumor experiment.

### Example 1

Solution of the sodium salt of D-myo-inositol-1,2,6-trisphosphate (IP₃) for injection.

0.5 g of the sodium salt of IP₃ and 0.77 g sodium chloride were dissolved in 98.73 ml of water for injection to form a solution suitable for injection into a person or an animal.

### Example 2

Rat glial cells were grown in a stationary suspension culture in a medium supplemented with 15 % heat inactivated horse serum and 2.5 % heat inactivated fetal calf serum in a 5 % CO₂ humidified atmosphere at 37°C.

The cells were harvested using trypsine solution and the concentration of cells were measured. The cells were centrifuged and resuspended to a concentration of 1 million cells/ml.

Twelve female specific pathogen free mice at an age of 12 weeks (weighing 20-25 g) were maintained in autoclaved cages with filter tops in horizontal air flow cabiners in a unit equipped with specific filters. Autoclaved bedding, irradiated feed and sterile water was used. The temperature and humidity was kept constant at 22 ± 2°C, 65 ± 5 % relative humidity.

To each animal 0.1 ml of the cellsuspension was inoculated subcutanousely in the right flank at day 0.

After five days, osmotic minipumps (ALZET) were surgically placed subcutanously into 7 animals, while 5 animals, shamoperated, served as a control group.

Each minipump contained approximately 200 µl of a solution of 1.0 g/2,5 ml of the sodium salt of 1-D-myo-inositol-1,2,6-trisphosphate (IP₃). The minipumps gave a continous dosage of IP₃ for 14 days.

The injection of glioma cells into the animals cause a growth of tumors. The growth of the tumors was examined by measuring two diameters along two perpendicular axes. The true tumor diameter was estimated as the diameter minus 0.5 mm (which is the thickness of the skin). Tumor area (A) was calculated by multiplying the two true diameters and tumor volume was calculated by the formula: /6 x A^{3/2} x 0.67. The obtained tumor volumes are shown in the following table:

| Tumor volume in mm³ | | |
|---|---|---|
| Day | IP₃-treatment | Control |
| 0 | 0 | 0 |
| 10 | 115 | 230 |
| 13 | 270 | 580 |
| 15 | 620 | 1190 |
| 17 | 880 | 2230 |
| 20 | 2420 | 4270 |

The results show a very marked effect of IP₃ to counteract the growth of tumor tissue induced by glioma cells.

### Example 3

In a procedure similar to the one described in example 2 prostate tumor growth was induced by subcutanous injection of a specific cell type to mice. One group of animals received IP₃ via osmotic minipumps while another group of animals served as a control. The tumor growth was examined and the tumor volume is shown in the following table:

| Tumor volume in mm³ | | |
|---|---|---|
| Day | IP₃-treatment | Control |
| 0 | 0 | 0 |
| 7 | 36 | 52 |
| 10 | 62 | 130 |
| 17 | 51 | 481 |
| 24 | 95 | 638 |
| 28 | 171 | 1016 |
| 35 | 215 | 1132 |

In this example the administration of IP₃ reduced the growth of prostate tumor tissue in a significant way.

### Example 4

Lewis lung carcinoma cells (ATCC CRL-1642) were grown as a suspension culture in a medium with 4.5 g/l glucose with 10 % heat inactivated bovine serum in a 5 % CO₂ humidified atmosphere at 37°C. The cells were subcultured by diluting cell suspensions 1:6. After harvesting by centrifugation and resuspension, the cells were counted in a counting chamber. The cells were resuspended with medium to a concentration of 10 million cells/ml.

Ten male specific pathogen free mice at an age of 6 weeks were maintained in autoclaved caves with filter tops in a barrier unit equipped with HEPA filters. Autoclaved bedding, irradiated feed and sterile filtered water was used. The temperature and humidity was kept constant (22 ± 2°C, 65 ± 5 % RH).

To each animal 0.1 ml of the cell suspension was inoculated subcutanousely in the right flank at day 0. After 15 days, osmotic minipumps (ALZET) were surgically placed subcutaneously into 5 animals while another 5 animals, sham operated, served as a control group.

Each minipump contained 226 µl of a solution of 0.4 g/ml of the sodium salt of 1-D-myo-inositol-1,2,6-trisphosphate (IP₃). The minipumps gave a continous dosage of IP₃ for 6 days.

The injection of lung carcinoma cells cause a formation of tumors and angiogenesis in lung tissue. The weight of the lungs after the experiment is considered as a measurement of the amount of tumor tissue which is formed.

In the sham operated animals, the mean lung weight was 0.312 g while the animals treated with IP₃ has a mean lung weight of 0.198 g. Furthermore, histological examination showed lower angiogenesis in the animals treated with IP₃. Thus the treatment with IP₃ reduces the tumor tissue and diminishes angiogenesis.

## Claims

1. The use of D-myo-inositol-1,2,6-trisphosphate for preparing a medicament with antimetastatic effects.

2. The use of D-myo-inositol-1,2,6-trisphosphate for preparing a medicament for preventing, alleviating or combatting tumor tissue growth and angiogenesis of tumor tissue.

3. The use according to claims 1 or 2 for the treatment of glioma.

4. The use according to claim 2 for the treatment of prostate carcinoma.

5. The use according to claim 2 for the treatment of lung carcinoma.

6. The use according to claim 2 for the treatment of melanoma.

7. The use according to any one of claims 1-6 wherein said compound is a salt of sodium, potassium, calcium, zinc, magnesium or a mixture thereof.

8. The use according to any one of claims 1-6 wherein D-myo-inositol-1,2,6-trisphosphate or a pharmaceutically acceptable salt thereof is used for preparing a medicament in a unit dosage form comprising tablets, granules, solutions or suspensions.

## Patentansprüche

1. Verwendung von D-myo-inositol-1,2,6-trisphosphat zur Herstellung eines Medikaments mit Wirkung gegen Metastasen.

2. Verwendung von D-myo-inositol-1,2,6-trisphosphat zur Herstellung eines Medikaments zum Verhindern, Lindern oder Bekämpfen von Tumorgewebewachstum und der Angiogenese von Tumorgewebe.

3. Verwendung gemäss Anspruch 1 oder 2, für die Behandlung von Gliom.

4. Verwendung gemäss Anspruch 2 für die Behandlung von Prostatakrebs.

5. Verwendung gemäss Anspruch 2 für die Behandlung von Lungenkrebs.

6. Verwendung gemäss Anspruch 2 für die Behandlung von Melanom.

7. Verwendung gemäss einem der Ansprüche 1 bis 6, wobei die Verbindung ein Natrium-, Kalium-, Calcium-, Zinkoder Magnesiumsalz oder eine Mischung davon ist.

8. Verwendung gemäss einem der Ansprüche 1 bis 6, wobei D-myo-inositol-1,2,6-trisphosphat oder ein pharmazeutisch annehmbares Salz davon zur Herstellung eines Medikaments in Einheitsdosisform, umfassend Tabletten, Körnchen, Lösungen oder Suspensionen, verwendet wird.

## Revendications

1. L'utilisation de D-myo-inositol-1,2,6-triphosphate pour préparer un médicament avec des effets anti-métastatiques.

2. L'utilisation de D-myo-inositol-1,2,6-triphosphate pour préparer un médicament afin de prévenir, soulager ou combattre la croissance de tissu tumoral et l'angiogénèse de tissu tumoral.

3. L'utilisation selon les revendications 1 et 2 pour le traitement d'un gliome.

4. L'utilisation selon la revendication 2 pour le traitement d'un carcinome de la prostate.

5. L'utilisation selon la revendication 2 pour le traitement d'un carcinome des poumons.

6. L'utilisation selon la revendication 2 pour le traitement d'un mélanome.

7. L'utilisation selon n'importe laquelle des revendications 1-6 où ledit composé est un sel de sodium, de potassium, de calcium, de zinc, de magnésium ou un mélange de ceux-ci.

8. L'utilisation selon n'importe laquelle des revendications 1-6 où le D-myo-inositol-1,2,6-triphosphate ou un sel de celui-ci acceptable du point de vue pharmaceutique est utilisé afin de préparer un médicament sous forme de dose unitaire comprenant des comprimés, des granulés, des solutions ou des suspensions.
